# EUROPEAN PATENT SPECIFICATION

(11) **EP 1 855 711 B1**
(45) Date of publication and mention of the grant of the patent: **17.06.2009**
(21) Application number: 06734022.4
(22) Date of filing: 27.01.2006
(51) Int. Cl.: A61K 38/19

(54) **USE OF BAFF TO TREAT Th2-MEDIATED CONDITIONS**
VERWENDUNG VON BAFF ZUR BEHANDLUNG Th2-INDUZIERTER LEIDEN
UTILISATION DE BAFF POUR TRAITER DES PATHOLOGIES INDUITES PAR Th2

(30) Priority: 28.01.2005 US 648098 P
(43) Date of publication of application: 21.11.2007
(73) Proprietor: Biogen Idec MA Inc., Cambridge, MA 02142 (US); The Garvan Institute of Medical Research, Darlinghurst Sydney NSW 2010 (AU)
(72) Inventor: MACKAY, Charles, Vaucluse, New South Wales, 2030 (AU); MACKAY, Fabienne, Vaucluse, New South Wales, 2030 (AU); KALLED, Susan, Concord, Massachusetts 01742 (US)
(74) Representative: Vossius & Partner
(86) International application number: PCT/US2006/003127
(87) International publication number: WO 2006/081516

(56) References cited:
- SUTHERLAND ANDREW P R ET AL: "BAFF augments certain Th1-associated inflammatory responses" JOURNAL OF IMMUNOLOGY, vol. 174, no. 9, May 2005 (2005-05), pages 5537-5544, XP002419137 ISSN: 0022-1767
- HUARD B ET AL: "T cell costimulation by the TNF ligand BAFF." JOURNAL OF IMMUNOLOGY (BALTIMORE, MD. : 1950) 1 DEC 2001, vol. 167, no. 11, 1 December 2001 (2001-12-01), pages 6225-6231, XP002419138 ISSN: 0022-1767
- THIEN MARILYN ET AL: "Excess BAFF rescues self-reactive B cells from peripheral deletion and allows them to enter forbidden follicular and marginal zone niches" IMMUNITY, vol. 20, no. 6, June 2004 (2004-06), pages 785-798, XP002419139 ISSN: 1074-7613
- HUARD B ET AL: "BAFF production by antigen-presenting cells provides T cell co-stimulation" INTERNATIONAL IMMUNOLOGY, OXFORD UNIVERSITY PRESS, GB, vol. 16, no. 3, March 2004 (2004-03), pages 467-475, XP002993868 ISSN: 0953-8178
- MACKAY FABIENNE ET AL: "The role of the BAFF/APRIL system in B cell homeostasis and lymphoid cancers" CURRENT OPINION IN PHARMACOLOGY, vol. 4, no. 4, August 2004 (2004-08), pages 347-354, XP002419140 ISSN: 1471-4892
- LITINSKIY MIKHAIL B ET AL: "DCs induce CD40-independent immunoglobulin class switching through BLyS and APRIL" NATURE IMMUNOLOGY, vol. 3, no. 9, September 2002 (2002-09), pages 822-829, XP002419141 ISSN: 1529-2908
- KALLED SUSAN L: "The role of BAFF in immune function and implications for autoimmunity" IMMUNOLOGICAL REVIEWS, vol. 204, no. April, April 2005 (2005-04), pages 43-54, XP002419142 ISSN: 0105-2896

### SEQUENCE LISTING

<110> Biogen Idec MA Inc.
   Garvan Institute of Medical Research
   Mackay, Charles
   Mackay, Fabienne
   Kalled, Susan
<120> USE OF BAFF TO TREAT Th2-MEDIATED CONDITIONS
<130> P0635
<150> 60/648,098
   <151> 2005-01-28
<160> 3
<170> PatentIn version 3.3
<210> 1
   <211> 285
   <212> PRT
   <213> Homo sapiens
<400> 1
   Met Asp Asp Ser Thr Glu Arg Glu Gln Ser Arg Leu Thr Ser Cys Leu
   1 5 10 15
   Lys Lys Arg Glu Glu Met Lys Leu Lys Glu Cys Val Ser Ile Leu Pro
   20 25 30
   Arg Lys Glu Ser Pro Ser Val Arg Ser Ser Lys Asp Gly Lys Leu Leu
   35 40 45
   Ala Ala Thr Leu Leu Leu Ala Leu Leu Ser Cys Cys Leu Thr Val Val
   50 55 60
   Ser Phe Tyr Gln Val Ala Ala Leu Gln Gly Asp Leu Ala Ser Leu Arg
   65 70 75 80
   Ala Glu Leu Gln Gly His His Ala Glu Lys Leu Pro Ala Gly Ala Gly
   85 90 95
   Ala Pro Lys Ala Gly Leu Glu Glu Ala Pro Ala Val Thr Ala Gly Leu
   100 105 110
   Lys Ile Phe Glu Pro Pro Ala Pro Gly Glu Gly Asn Ser Ser Gln Asn
   115 120 125
   Ser Arg Asn Lys Arg Ala Val Gln Gly Pro Glu Glu Thr Val Thr Gln
   130 135 140
   Asp Cys Leu Gln Leu Ile Ala Asp Ser Glu Thr Pro Thr Ile Gln Lys
   145 150 155 160
   Gly Ser Tyr Thr Phe Val Pro Trp Leu Leu Ser Phe Lys Arg Gly Ser
   165 170 175
   Ala Leu Glu Glu Lys Glu Asn Lys Ile Leu Val Lys Glu Thr Gly Tyr
   180 185 190
   Phe Phe Ile Tyr Gly Gln Val Leu Tyr Thr Asp Lys Thr Tyr Ala Met
   195 200 205
   Gly His Leu Ile Gln Arg Lys Lys Val His Val Phe Gly Asp Glu Leu
   210 215 220
   Ser Leu Val Thr Leu Phe Arg Cys Ile Gln Asn Met Pro Glu Thr Leu
   225 230 235 240
   Pro Asn Asn Ser Cys Tyr Ser Ala Gly Ile Ala Lys Leu Glu Glu Gly
   245 250 255
   Asp Glu Leu Gln Leu Ala Ile Pro Arg Glu Asn Ala Gln Ile Ser Leu
   260 265 270
   Asp Gly Asp Val Thr Phe Phe Gly Ala Leu Lys Leu Leu
   275 280 285
<210> 2
   <211> 309
   <212> PRT
   <213> Mus musculus
<400> 2
   Met Asp Glu Ser Ala Lys Thr Leu Pro Pro Pro Cys Leu Cys Phe Cys
   1 5 10 15
   Ser Glu Lys Gly Glu Asp Met Lys Val Gly Tyr Asp Pro Ile Thr Pro
   20 25 30
   Gln Lys Glu Glu Gly Ala Trp Phe Gly Ile Cys Arg Asp Gly Arg Leu
   35 40 45
   Leu Ala Ala Thr Leu Leu Leu Ala Leu Leu Ser Ser Ser Phe Thr Ala
   50 55 60
   Met Ser Leu Tyr Gln Leu Ala Ala Leu Gln Ala Asp Leu Met Asn Leu
   65 70 75 80
   Arg Met Glu Leu Gln Ser Tyr Arg Gly Ser Ala Thr Pro Ala Ala Ala
   85 90 95
   Gly Ala Pro Glu Leu Thr Ala Gly Val Lys Leu Leu Thr Pro Ala Ala
   100 105 110
   Pro Arg Pro His Asn Ser Ser Arg Gly His Arg Asn Arg Arg Ala Phe
   115 120 125
   Gln Gly Pro Glu Glu Thr Glu Gln Asp Val Asp Leu Ser Ala Pro Pro
   130 135 140
   Ala Pro Cys Leu Pro Gly Cys Arg His Ser Gln His Asp Asp Asn Gly
   145 150 155 160
   Met Asn Leu Arg Asn Ile Ile Gln Asp Cys Leu Gln Leu Ile Ala Asp
   165 170 175
   Ser Asp Thr Pro Thr Ile Arg Lys Gly Thr Tyr Thr Phe Val Pro Trp
   180 185 190
   Leu Leu Ser Phe Lys Arg Gly Asn Ala Leu Glu Glu Lys Glu Asn Lys
   195 200 205
   Ile Val Val Arg Gln Thr Gly Tyr Phe Phe Ile Tyr Ser Gln Val Leu
   210 215 220
   Tyr Thr Asp Pro Ile Phe Ala Met Gly His Val Ile Gln Arg Lys Lys
   225 230 235 240
   Val His Val Phe Gly Asp Glu Leu Ser Leu Val Thr Leu Phe Arg Cys
   245 250 255
   Ile Gln Asn Met Pro Lys Thr Leu Pro Asn Asn Ser Cys Tyr Ser Ala
   260 265 270
   Gly Ile Ala Arg Leu Glu Glu Gly Asp Glu Ile Gln Leu Ala Ile Pro
   275 280 285
   Arg Glu Asn Ala Gln Ile Ser Arg Asn Gly Asp Asp Thr Phe Phe Gly
   290 295 300
   Ala Leu Lys Leu Leu
   305
<210> 3
   <211> 151
   <212> PRT
   <213> Artificial
<220>
   <223> Consensus BAFF sequence
<220>
   <221> misc_feature
   <222> (7)..(9)
   <223> Xaa can be any naturally occurring amino acid
<220>
   <221> misc_feature
   <222> (20)..(20)
   <223> Xaa can be any naturally occurring amino acid
<220>
   <221> misc_feature
   <222> (25)..(25)
   <223> Xaa can be any naturally occurring amino acid
<220>
   <221> misc_feature
   <222> (28)..(28)
   <223> Xaa can be any naturally occurring amino acid
<220>
   <221> misc_feature
   <222> (42)..(42)
   <223'> Xaa can be any naturally occurring amino acid
<220>
   <221> misc_feature
   <222> (52)..(52)
   <223> Xaa can be any naturally occurring amino acid
<220>
   <221> misc_feature
   <222> (54)..(55)
   <223> Xaa can be any naturally occurring amino acid
<220>
   <221> misc_feature
   <222> (63)..(63)
   <223> Xaa can be any naturally occurring amino acid
<220>
   <221> misc_feature
   <222> (70)..(72)
   <223> Xaa can be any naturally occurring amino acid
<220>
   <221> misc_feature
   <222> (77)..(77)
   <223> Xaa can be any naturally occurring amino acid
<220>
   <221> misc_feature
   <222> (104)..(104)
   <223> Xaa can be any naturally occurring amino acid
<220>
   <221> misc_feature
   <222> (118)..(118)
   <223> Xaa can be any naturally occurring amino acid
<220>
   <221> misc_feature
   <222> (125)..(125)
   <223> Xaa can be any naturally occurring amino acid
<220>
   <221> misc_feature
   <222> (138)..(139)
   <223> Xaa can be any naturally occurring amino acid
<220>
   <221> misc_feature
   <222> (142)..(142)
   <223> Xaa can be any naturally occurring amino acid
<400> 3
   Gln Gly Pro Glu Glu Thr Xaa Xaa Xaa Gln Asp Cys Leu Gln Leu Ile
   1               5                   10                  15
   Ala Asp Ser Xaa Thr Pro Thr Ile Xaa Lys Gly Xaa Thr Thr Phe Val
               20                  25                  30
   Pro Trp Leu Leu Ser Phe Lys Arg Gly Xaa Ala Leu Glu Glu Lys Glu
           35                  40                  45
   Asn Lys Ile Xaa Val Xaa Xaa Thr Gly Thr Phe Phe Ile Tyr Xaa Gln
       50                  55                  60
   Val Leu Tyr Thr Asp Xaa Xaa Xaa Ala Met Gly His Xaa Ile Gln Arg
   65                  70                  75                  80
   Lys Lys Val His Val Phe Gly Asp Glu Leu Ser Leu Val Thr Leu Phe
                   85                  90                  95
   Arg Cys Ile Gln Asn Met Pro Xaa Thr Leu Pro Asn Asn Ser Cys Tyr
               100                 105                 110
   Ser Ala Gly Ile Ala Xaa Leu Glu Glu Gly Asp Glu Xaa Gln Leu Ala
           115                 120                 125
   Ile Pro Arg Glu Asn Ala Gln Ile Ser Xaa Xaa Gly Asp Xaa Thr Phe
       130                 135                 140
   Phe Gly Ala Leu Lys Leu Leu
   145                 150

## Description

### BACKGROUND OF THE INVENTION

CD4+ T cells can be divided into two major subsets, Th1 and Th2, based upon the specific cytokines produced and the functional activities exhibited by each cell type. Th1 cells produce interferon-g (IFN-g), interleukin-12 (IL-12), and lymphotoxin (LT), which promote macrophage activation and the generation of cell-mediated immunity. Th2 cells produce a variety of cytokines including IL-4, IL-5, IL-10, and IL13, and provide help for the maturation of B cells to immunoglobulin-secreting cells, thereby primarily activating humoral defense mechanisms. Homeostasis between Th1 and Th2 activity is required for immune regulation and an imbalance in Th1/Th2 responses can contribute to certain pathological conditions. Strong and persistent Th1 responses are involved in organ-specific autoimmunity, for example. In contrast, polarized Th2 responses favor a reduced protection against certain viral infections (including HIV) and are responsible for triggering of allergic atopic disorders, such as asthma.

### SUMMARY OF THE INVENTION

The invention is based, in part, on the discovery of a role for BAFF as an inhibitor of Th2 responses and a potentiator of Th1 responses.

Accordingly, in one aspect, the invention provides compositions and methods for treating Th2-mediated pathogenic conditions, e.g., asthma, allergic rhinitis, allergy, eczema, and other atopic conditions. The methods include administering to a mammal, preferably a human, having a Th2-mediated condition, a BAFF polypeptide The method includes administering a soluble BAFF, e.g., a pharmaceutical composition containing soluble BAFF, in an amount and for a time sufficient to treat the condition.

In one embodiment, the condition is allergic asthma. In this embodiment, the method optionally also includes identifying a subject who is at risk for, or has, allergic asthma. Optionally, the method also includes evaluating a symptom of asthma in the subject, e.g., IgE levels, airway hyperresponsiveness, coughing, wheezing, chest tightness, dyspnea, airway smooth muscle contraction, bronchial mucus secretion, inflammation, vasodilation, recruitment of inflammatory cells (e.g., neutrophils, monocytes, macrophages, lymphocytes, eosinophils), goblet cell hyperplasia, release of inflammatory mediators by mast cells or migrating inflammatory cells. The evaluation step can be performed before, during and/or after the administration step. The evaluation can be performed by a physician, other health care provider or by the subject. The evaluation can be performed one or more times, e.g., one or more times after administration, e.g., at least twice during a one week, one month, two month, three month, six month period after the administration, or longer.

In a preferred embodiment, the method includes determining whether the administration of the soluble BAFF (or multiple administrations) reduced the severity or initiation of one or more symptoms of airway disease in the subject.

In some embodiments, the soluble BAFF is co-administered with a second agent effective to treat asthma in the subject, e.g., a corticosteroid, bronchodilator, leukotriene modifier, anti-inflammatory agent. "Co-administered" or "administered in combination" means administration at the same time or within an interval, e.g., a week, such that the effects of the substances on the patient overlap.

In another embodiment, the condition is allergy, e.g., food allergy or seasonal (e.g., pollen) allergy. A diagnosis of allergy may be made by one or more of: administration of an allergen skin test; determination of IgE concentration in serum (e.g., IgE>300 ng/ml); and determination of allergen-specific IgE or IgG antibodies in serum.

As used herein, "soluble BAFF" is a polypeptide that includes a sequence at least 85% identical (preferably, at least 90%, 95%, 98%, 99%, or 100% identical) to the TNF-like domain of BAFF, and lacks a transmembrane domain. In certain embodiments, the soluble BAFF is a polypeptide including all or a substantial part (e.g., at least 85%, 90%, 95% or greater) of the TNF-like domain of BAFF.

Soluble BAFF can be administered in one or more of the following periods: prior to an atopic subject's exposure to allergen; after exposure to allergen but prior to the onset of symptoms; at the time of onset of symptoms; after onset of symptoms.

In one embodiment, soluble BAFF is administered as a course of treatment, e.g., in periodic administrations of predetermined frequency, e.g., daily, weekly, biweekly or monthly. In some embodiments, soluble BAFF can be administered for a period of time and/or in an amount sufficient to reduce (e.g., to substantially reduce) the frequency or severity of episodes of wheezing, coughing, shortness of breath, or tightness in the chest, e.g., over a period of time, e.g., 3 months, 6 months, a year or more.

In embodiments described hereinabove, a BAFF agonist, e.g., an anti-BAFF or anti-BAFF-R (BR3) agonist antibody, can be used in addition to a soluble BAFF.

In another aspect, the invention features the use of BAFF, e.g., soluble BAFF, in a method for treating a subject who has an inadequate Th1 response. For example, the subject may have inadequate responses to certain viral or bacterial infections, and increased Th1 responses would aid in the production of cytokines such as IFN gamma and clearance of the pathogen. Enhanced Th1 responses may also promote anti-tumor defense and cytotoxicity of cancerous cells.

In another aspect, the invention features the use of BAFF, e.g., soluble BAFF, in a method for treating a subject who has a microbial infection, e.g., infection with a pathogen, e.g., a viral or intracellular bacterial pathogen, e.g., a subject who has hepatitis (e.g., hepatitis B), schistosomiasis, cryptococcal infection. In some embodiment, the methods described herein can be used to treat subjects in an epidemic or pandemic situation such as flu, or SARS, where individuals have succumbed to infection because of an inability of their immune system to respond to the viral infection.

In one embodiment, the subject has an inadequate Th1 response prior to administration of BAFF.

In embodiments described hereinabove, a BAFF agonist, e.g., an anti-BAFF or anti-BAFF-R (BR3) agonist antibody, can be used in addition to a soluble BAFF.

As used herein, the terms "to treat," "treating," and "treatment" refer to administering a therapy in an amount, manner, and/or mode effective to improve or ameliorate a symptom or parameter that characterizes a pathological condition; to reduce the severity of a symptom or parameter that characterizes a pathological condition; to prevent, slow or reverse progression of the pathological condition; or to prevent one or more symptom or parameter of the pathological condition.

The foregoing summary and the following description are not restrictive of the invention as claimed.

### BRIEF DESCRIPTION OF THE FIGURES

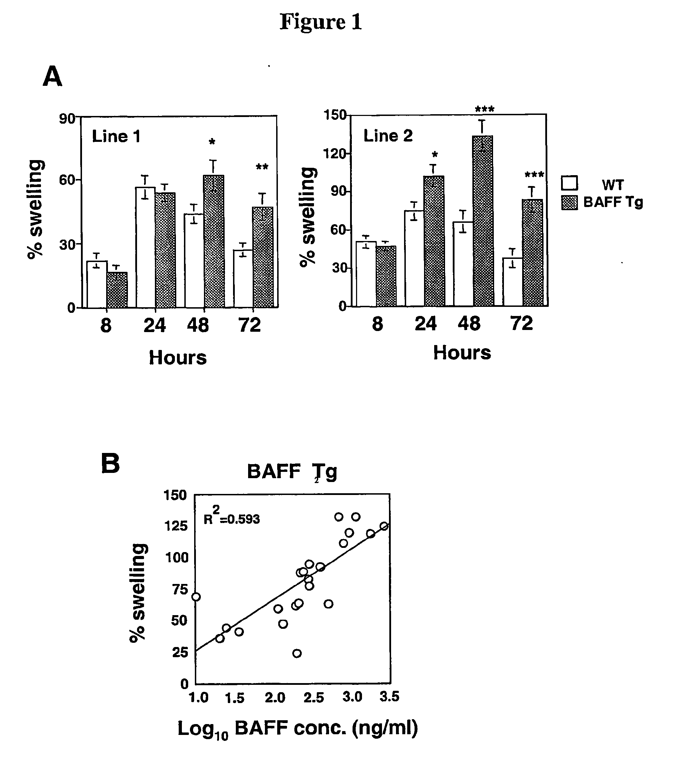

**Figure 1** is a set of graphs showing that BAFF Tg mice have increased DTH responses. A, Increased footpad swelling over time in 2 lines of BAFF Tg mice (n=6, line 2; n=10 line 1). B, Serum levels of BAFF in BAFF Tg mice were measured using ELISA and plotted against footpad swelling at 48 h following DTH.

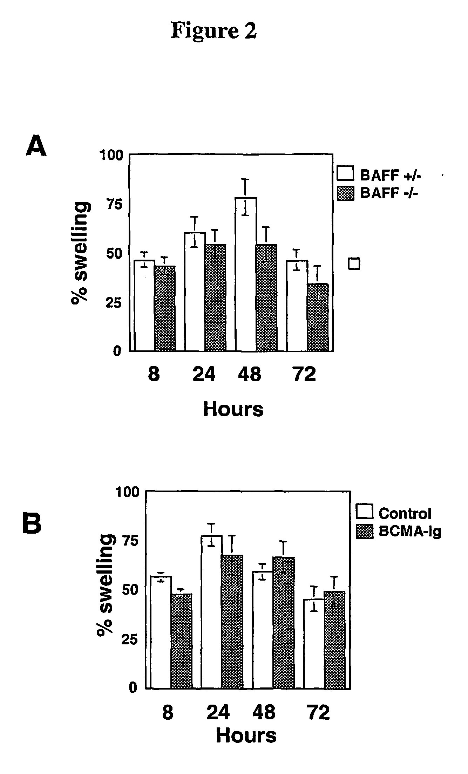

**Figure 2** is a set of graphs showing DTH responses. A, DTH responses in BAFF-/- mice, and mice heterozygous for the knockout allele (n=6); B, C57B6 mice treated with BCMA-Ig showed no reduction in DTH severity compared to control (PBS) treated mice (n=6).

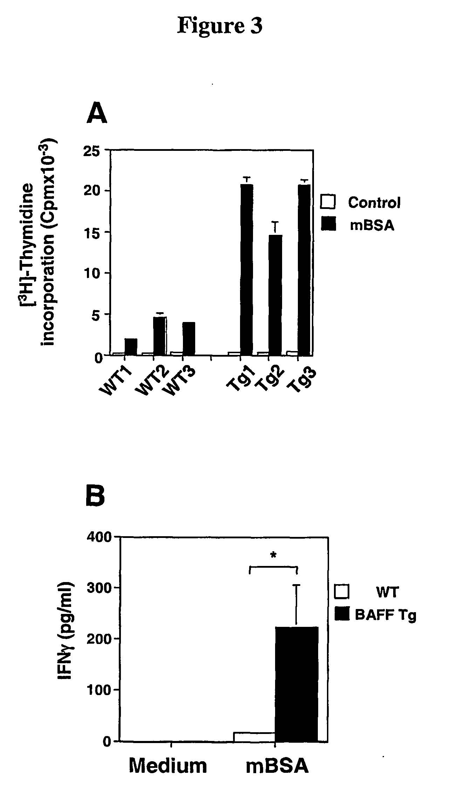

**Figure 3** is a set of graphs showing increased mBSA specific in vitro recall responses by T cells in BAFF Tg mice. A, Mice were injected at the tailbase with 250 ug of mBSA in CFA, and inguinal lymph nodes were collected 7 days later. Cultures were normalised to 2x10⁵ T cells/well and cultured in triplicate with medium alone or 40 ug/ml mBSA for 72 h. Proliferation was measured by thymidine uptake. B, IFN-gamma levels from culture supernatants were measured by ELISA (n=3).

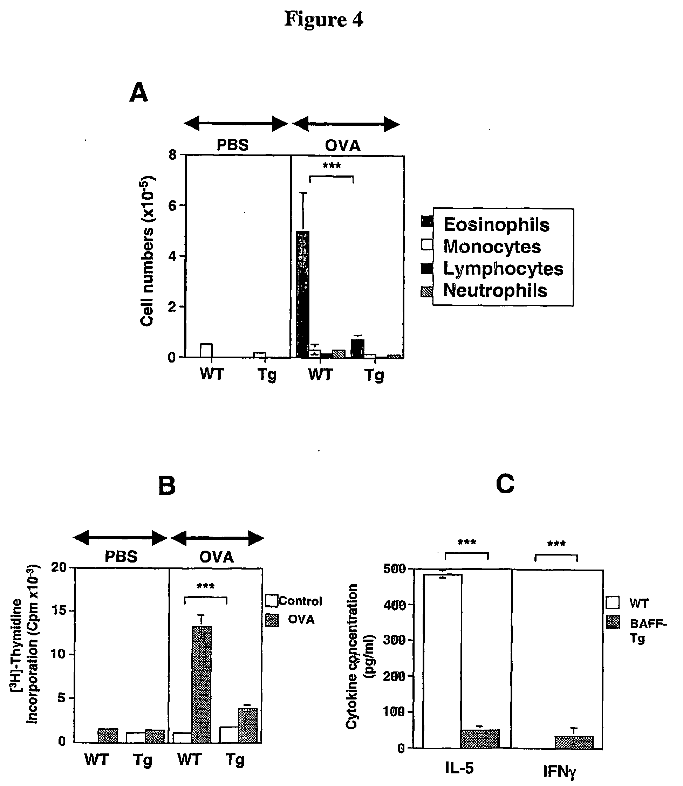

**Figure 4** shows suppression of allergic airway inflammation in BAFF Tg mice. A, BAL fluid was recovered from BAFF Tg mice and wildtype controls after an 8 day aerosol exposure regime, and constituent cell types determined by cytospin and Giemsa staining (n=5). B, Peri-bronchial lymph nodes were collected and pooled. Cultures were normalised to 2x10⁵ T cells/well and were cultured in triplicate with media alone or 100ug/ml OVA for 72 h, and proliferation was measured by thymidine uptake. C, IL-5 and IFN-gamma levels from culture supernatants were measure by ELISA.

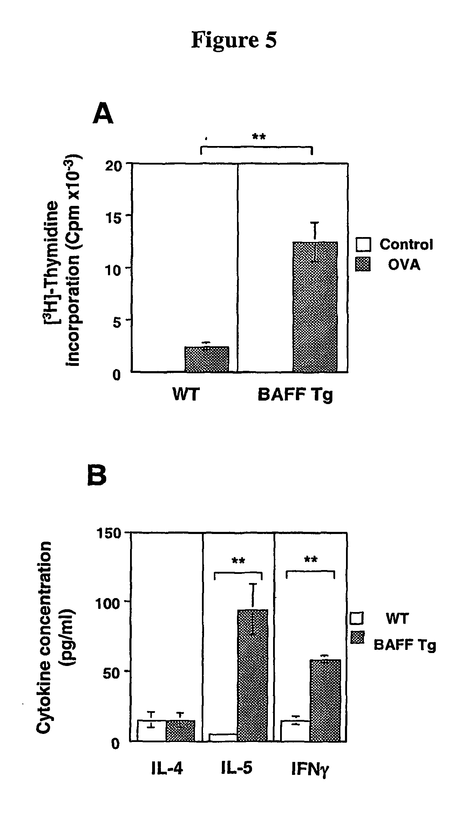

**Figure 5** shows increased OVA specific in vitro recall responses in BAFF Tg. A, Mice were injected at the tailbase with 100ug of OVA in alum and inguinal lymph nodes collected 7 days later (n=4). Cultures were normalised to 2x10⁵ T cells/well and were cultured in triplicate with medium alone or 100ug/ml OVA for 72 h. Proliferation was measured by thymidine uptake. B, Cytokine levels from culture supernatants were measured by ELISA.

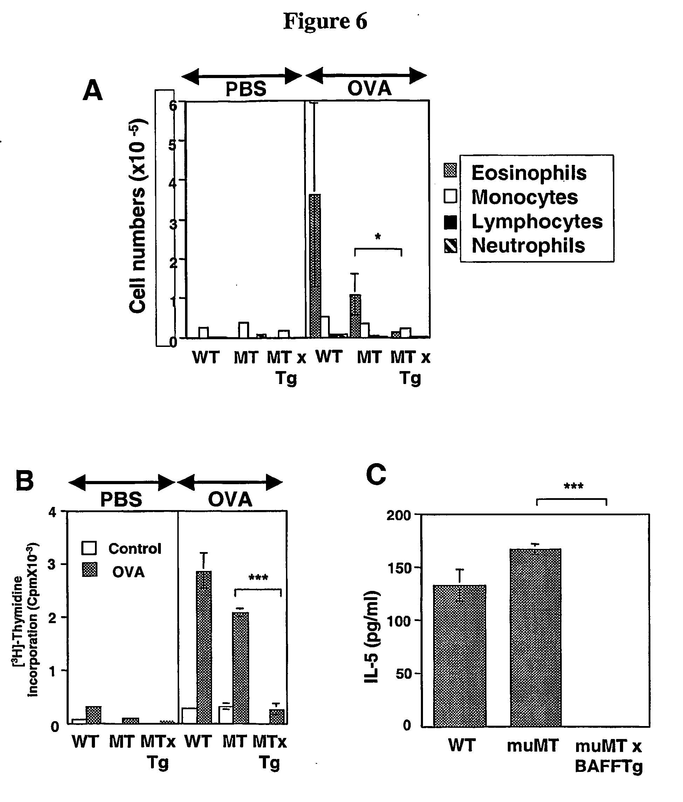

**Figure 6** shows suppression of allergic airway inflammation in BAFF Tg mice is B cell independent. A, BAL fluid was recovered from WT, uMT and uMT x BAFF Tg mice after aerosol exposure, and constituent cell types determined by cytospin and Giemsa staining (n=5). B, Peri-bronchial lymph nodes were collected and pooled into groups. Cultures were normalised to 2x10⁵ T cells/well and restimulated with media alone or 100ug/ml OVA for 72 h, with proliferation measured by thymidine uptake. C, IL-5 and IFN-gamma levels from culture supernatants were measure by ELISA.

### DETAILED DESCRIPTION OF THE INVENTION

B cell activating factor belonging to the TNF family (BAFF, BLyS) is a critical regulator of B cell maturation and survival, and its over-expression in BAFF transgenic (Tg) mice results in the development of autoimmune disease. BAFF also affects T cell function, through binding to one of the BAFF receptors, BAFF-R.

As described herein, BAFF over-expression in vivo promotes the DTH reaction, which is a classical Th1 response, and suppresses Th2 dependent allergic airway responses. The effects of BAFF on the T cell system were profound, in terms of subset ratios and Th1 or Th2 responses. In a Th2 model of allergic airway inflammation, BAFF Tg mice were largely protected from inflammation. Eosinophil infiltration into the lung was reduced along with significantly reduced OVA-specific T cell proliferation and IL-5 production. Crossing BAFF Tg mice onto the B-cell deficient µMT background demonstrated that the protection provided by BAFF in allergic airway inflammation was B cell independent. Thus, local and/or systemically distributed BAFF affects Th1 and Th2 responses, and can impacts on the course of T cell-mediated inflammatory reactions. These findings demonstrate a novel role for BAFF in vivo as an inhibitor of Th2 responses, and illustrate the importance of BAFF for T cell as well as B cells responses.

US Published Application Nos. 2003/0059937 and 2003/0091565 suggest that agents that antagonize BAFF (e.g., anti-BAFF antibodies or BAFF-blocking peptides) may be useful to treat asthma. The data described herein show that administering, rather than blocking, BAFF inhibits Th2 responses and can thus be used to treat Th2 related conditions, such as asthma.

BAFF Compositions

BAFF (also known as BLyS, TALL-1, THANK, zTNF4, neutrokine a, NTN2, TNSF13b, Kay ligand, and MARCH) is one of the recently discovered members of the TNF family. Briefly, BAFF has been implicated in costimulation of B cells (Moore et al. (1999) Science, 285:260-263; Schneider et al. (1999) J. Exp. Med., 189:1747-1756; Mukhopadhyay et al. (1999) J. Biol. Chem., 274:15978-15981); increased B cell proliferation (Moore et al. (1999) Science, 285:260-263); and increased survival of normally deleted B cells (Khare et al. (2000) Proc. Natl. Acad. Sci. USA, 97:3370-3375; Gross et al. (2000) Nature, 404:995-999; Mackay et al. (1999) J. Exp. Med., 190:1697-1710). For a review on BAFF, see, e.g., Mackay et al. (2002) Nature Reviews: Immunology, 2:465-475; and Kalled et al. (2003) Expert Opin. Ther. Targets, 7(1):115-123 and OMIM entry No. 603969.

The amino acid and nucleic acid sequences of naturally occurring full-length human BAFF are available under GenBank™ accession Nos. Q9Y275 (SEQ ID NO:1) and AF136293. The amino acid and nucleic acid sequences of full-length mouse BAFF are available under GenBank™ accession Nos. Q9WU72 (SEQ ID NO:2) and AF119383. Full-length BAFF is a type II membrane protein having intracellular, transmembrane, and extracellular domains. In human BAFF, these domains are comprised approximately of amino acids 1-46, 47-73, and 74-285 of SEQ ID NO:1, respectively. In mouse BAFF, these domains are comprised approximately of amino acids 1-53, 53-73, 74-309 of SEQ ID NO:2, respectively. A naturally occurring soluble form of BAFF exists, in which proteolytic cleavage occurs between amino acids R133 and A134 in human BAFF (amino acids R125 and A126 in mouse BAFF as predicted), resulting in a water-soluble biologically active C-terminal portion of BAFF.

Compositions suitable for use in methods of invention include soluble BAFF. Such soluble forms of BAFF generally do not comprise the transmembrane and intracellular domains. Since naturally occurring soluble BAFF does not comprise a portion of the extracellular domain (i.e., amino acids 74-133 of SEQ ID NO:1 or amino acids 74-125 of SEQ ID NO:2), soluble BAFF of the invention may likewise exclude these regions.

Within the extracellular domain, BAFF shares identity with other TNF family members: 28.7% with APRIL, 16.2% with TNF-α, and 14.1% with lymphotoxin(LT)-α. The extracellular domain of BAFF, including naturally occurring soluble BAFF, therefore contains the TNF-like domain delimited by amino acids 145-284 in SEQ ID NO:1 and amino acids 170-305 in SEQ ID NO:2. Accordingly, in certain embodiments, the soluble BAFF is a polypeptide comprising all or a substantial part of the TNF-like domain of BAFF, e.g., amino acids 145-284 of SEQ ID NO:1 (human BAFF), amino acids 170-305 of SEQ ID NO:2 (mouse BAFF), amino acids 11-150 of SEQ ID NO:3 (generic sequence), or a sequence at least 85%, 90%, or 95% identical to SEQ ID NO: 1 or SEQ ID NO:2. The TNF-like domain of human BAFF is greater than 85% identical to mouse BAFF.

Percent identity between two amino acid sequences may be determined by standard alignment algorithms such as, for example, Basic Local Alignment Tool (BLAST) described in Altschul et al. (1990) J. Mol. Biol., 215:403-410, the algorithm of Needleman et al. (1970) J. Mol. Biol., 28:444-453, or the algorithm of Meyers et al. (1988) Comput. Appl. Biosci., 4:11-17. Such algorithms are incorporated into the BLASTN, BLASTP, and "BLAST 2 Sequences" programs (see www.ncbi.nlm.nih.gov/BLAST). When utilizing such programs, the default parameters can be used. For example, for nucleotide sequences the following settings can be used for "BLAST 2 Sequences": program BLASTN, reward for match 2, penalty for mismatch -2, open gap and extension gap penalties 5 and 2 respectively, gap x_dropoff 50, expect 10, word size 11, filter ON. For amino acid sequences the following settings can be used for "BLAST 2 Sequences": program BLASTP, matrix BLOSUM62, open gap and extension gap penalties 11 and 1 respectively, gap x_dropoff 50, expect 10, word size 3, filter ON.

In nonlimiting illustrative embodiments, soluble BAFF comprises amino acids 134-285 of SEQ ID NO:1, or N- and/or C-terminal truncations thereof. For example, the N-terminus of soluble BAFF may be between residues 134-170 of SEQ ILL NO:1, e.g., the N-terminus of soluble BAFF may extend up to and include amino acid 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, or 170; while independently, the C-terminus be between residues 250-285 of SEQ ID NO:1, e.g., it may extend up to and include amino acid 285, 284, 283, 282, 281, 280, 279, 278, 277, 276, 275, 274, 273, 272, 271, 270, 269, 268, 267, 266, 265, 264, 263, 262, 261, 260, 259, 258, 257, 256, 255, 254, 253, 252, 251, 250 of SEQ ID NO:1. In one embodiment, soluble BAFF comprises amino acids 136-285 of SEQ ID NO:1.

In other nonlimiting illustrative embodiments, soluble BAFF comprises amino acids 126-309 of SEQ ID NO:2, or an N- and/or C-terminal truncations thereof. For example, the N-terminus of soluble BAFF may extend up to and include amino acid 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, or 170; while independently, the C-terminus may extend to and include amino acid 309, 308, 307, 306, 305, 304, 303, 302, 301, 300, 299, 298, 297, 296, 295, 294, 293, 292, 291, 290, 289, 288, 287, 286, 285, 284, 283, 282, 281, 280, 279, 278, 277, 276, 275, 274, 273, 272, 271, or 270 of SEQ ID NO:2.

Composition comprising soluble BAFF suitable for use in the methods of the invention further include such derivatives of BAFF in which the native BAFF sequence is mutated, partially deleted, and/or contains one or more insertions so long as changes to the native sequence do not substantially affect the biological activity of the molecule. Such changes may involve, for example, conservative amino acid substitution(s) according to Table 1. Nonlimiting examples of such changes are shown in SEQ ID NO:3. It is contemplated that soluble BAFF may contain no more than, for example, 50, 40, 30, 25, 20, 15, 10, 5, 4, 3, 2, or 1 amino acids that are substituted, deleted, or inserted relative to the naturally occurring BAFF sequences as set out from amino acid (aa) 136 to aa 285 of SEQ ID NO: 1 or from aa 126 to aa 309 of SEQ ID NO:2.

**Table 1**

| Original Residues | Exemplary Substitutions |
|---|---|
| **Ala (A)** | Val, Leu, Ile |
| **Arg (R)** | Lys, Gln, Asn |
| **Asn (N)** | Gln |
| **Asp (D)** | Glu |
| **Cys (C)** | Ser, Ala |
| **Gln (Q)** | Asn |
| **Gly (G)** | Pro, Ala |
| **His (H)** | Asn, Gln, Lys, Arg |
| **Ile (I)** | Leu, Val, Met, Ala, Phe, Norleucine |
| **Leu (L)** | Norleucine, Ile, Val, Met, Ala, Phe |
| **Lys (K)** | Arg, 1,4-Diamino-butyric Acid, Gln, Asn |
| **Met (M)** | Leu, Phe, Ile |
| **Phe (F)** | Leu, Val, Ile, Ala, Tyr |
| **Pro (P)** | Ala |
| **Ser (S)** | Thr, Ala, Cys |
| **Thr (T)** | Ser |
| **Trp (W)** | Tyr, Phe |
| **Tyr (Y)** | Trp, Phe, Thr, Ser |
| **Val (V)** | Ile, Met, Leu, Phe, Ala, Norleucine |

In some embodiments, soluble BAFF further comprises a heterologous amino acid sequence, e.g., a portion of one or more proteins other than BAFF, covalently bound to the BAFF portion at the latter's N- and/or C-terminus, and optionally further comprising a linker. The non-BAFF protein can be, for example, an immunoglobulin (e.g., the Fc portion of an immunoglobulin of any type or subtype (e.g., IgG (IgG₁, IgG₄), IgA, IgE, and IgM)), albumin, APRIL, or an affinity tag (e.g., myc-tag, His-tag). Soluble BAFF may also be linked to nonproteinaceous polymers, e.g., polyethylene glycol (PEG) and polypropylene glycol.

Additional soluble BAFF compositions suitable for use in the methods of the invention are described in, e.g., in United States Patents No. 6,689,579; 6,475,986; 6,297,736; United States Patent Application Publication Nos. 2003/0175208; 2002/0064829; 2003/0022239; 2003/0095967; 2002/0037852; 2002/0055624; 2001/0010925; 2003/0023038; 2003/0119149; 2003/0211509; PCT Application Publication Nos. WO 99/117791; WO 00/43032; WO 98/27114; WO 98/18921; WO 98/55620; WO 99/12964; WO 99/11791; WO 00/39295; WO 00/26244; WO 01/96528; WO 02/15930; WO 03/033658; WO 03/022877; WO 03/040307; WO 03/050134; WO 03/035846; WO 03/060072; WO 03/060071; WO 04/016737; WO 00/43032; WO 00/47740; WO 00/45836; and EPC Application Publication No. 1146892.

The biological activity of soluble forms of BAFF may be evaluated using one or more of the following assays: (1) B cell proliferation assay as described in, e.g., Scheider et al. (1999) J. Exp. Med., 189(11):1747-1756; (2) B cell survival assay as described in, e.g., Batten et al. (2000) J. Exp. Med., 192(10):1453-1465; (3) NFκB assay as described in, e.g., Claudio et al. (2002) Nature Imm.,3(10):898-899; (4) Ig secretion assay as described in, e.g., Moore et al. (1999) Science, 285:260-263; and (5) in vivo treatment of mice as described here or in, e.g., Moore et al. (1999) Science, 285:260-263.

The ability of BAFF to bind to one of its receptors (e.g., TACI, BCMA, BAFF-R) may optionally be used to pre-screen soluble BAFF forms before or in conjunction with evaluating their biological activity. Suitable receptor binding assays are described in, e.g., Gavin et al. (2003) J. Biol. Chem., 278(40):38220-38228. Accordingly, in some embodiments, soluble BAFF comprises a fragment of the BAFF extracellular domain capable of binding to a BAFF receptor. It is contemplated that such a fragment may comprise one or more regions of at least 20, 30, 40, 50, 60, 70, 80, 90, 100, 110, or 120 contiguous amino acids of SEQ ID NO:1, SEQ ID NO:2, SEQ ID NO:3, or soluble BAFF derived therefrom.

Methods of making soluble BAFF are well known in the art (see, e.g., references cited herein and Fernandez et al. (1999) Gene Expression Systems, Academic Press).

BAFF Agonists

BAFF agonists useful in the methods described herein include agonist antibodies, e.g., anti-BAFF or anti-BR3 agonist antibodies or functional, antigen-binding fragments thereof, which can mimic the effect of soluble BAFF. Methods of making therapeutic antibodies are known in the art (see, e.g., Methods in Molecular Medicine: Diagnostic and Therapeutic Antibodies, Catherine Urch and Andrew George (Eds.), Humana Press, 1st edition (August 15, 2000)). Agonist activity of an antibody can be assayed and determined using a BAFF activity assay, e.g., as described herein.

As used herein, the term "antibody" refers to a protein that includes at least one immunoglobulin variable region, e.g., an amino acid sequence that provides an immunoglobulin variable domain or an immunoglobulin variable domain sequence. For example, an antibody can include a heavy (H) chain variable region (abbreviated herein as VH), and a light (L) chain variable region (abbreviated herein as VL). In another example, an antibody includes two heavy (H) chain variable regions and two light (L) chain variable regions. The term "antibody" encompasses antigen-binding fragments of antibodies (e.g., single chain antibodies, Fab fragments, F(ab')2 fragments, Fd fragments, Fv fragments, and dAb fragments) as well as complete antibodies, e.g., intact and/or full length immunoglobulins of types IgA, IgG (e.g., IgG1, IgG2, IgG3, IgG4), IgE, IgD, IgM (as well as subtypes thereof). The light chains of the immunoglobulin may be of types kappa or lambda. An antibody may be glycosylated. An antibody can be functional for antibody-dependent cytotoxicity and/or complement-mediated cytotoxicity, or may be non-functional for one or both of these activities.

Cell and Gene Therapy

In one aspect, polynucleotides and vectors encoding the BAFF polypeptides described herein are administered either alone or in any combination using standard vectors and/or gene delivery systems, and, optionally, together with a pharmaceutically acceptable carrier or excipient. Subsequent to administration, the polynucleotides or vectors may be stably integrated into the genome of the subject, such as a human.

Alternatively, pharmaceutical compositions, including, e.g., vectors, such as viral vectors, of the invention are designed to be specific for (e.g., "target to") certain cells or tissues, e.g., the lung; they can also be designed to persist in cells. Suitable pharmaceutical carriers and excipients are well known in the art.

Furthermore, it is possible to use a pharmaceutical composition comprising a polynucleotide or vector encoding a BAFF polypeptide in gene therapy. Suitable gene delivery systems may include liposomes, receptor-mediated delivery systems, naked DNA, and viral vectors such as herpes viruses, retroviruses, adenoviruses, and adeno-associated viruses, among others. Delivery of nucleic acids to a specific site in the body for gene therapy may also be accomplished using a biolistic delivery system, such as that described by Williams (Proc. Natl. Acad. Sci. USA 88 (1991), 2726-2729). Further methods for the delivery of nucleic acids comprise particle-mediated gene transfer as, e.g., described in Verma, Gene Ther. 15 (1998), 692-699. Gene therapy approaches for targeting cells of the airway are described, e.g., in Johnson, Ann N Y Acad Sci 2001 December;953:43-52 and Davies et al., Curr Opin Pharmacol 2001 June; 1(3):272-7.

It is to be understood that the introduced polynucleotides and vectors express a gene product after introduction into the cell; they can remain in this status during the lifetime of the cell. For example, cell lines that stably express the polynucleotide under the control of appropriate regulatory sequences may be engineered according to methods well known to those skilled in the art. Rather than using expression vectors that contain viral origins of replication, host cells can be transformed with the polynucleotide of the invention and a selectable marker, either on the same or separate plasmids. Following the introduction of foreign DNA, engineered cells may be allowed to grow for about 1 to 2 days in an enriched media, and then are switched to a selective media. The selectable marker in the recombinant plasmid confers resistance to the selection. This allows for the selection of cells having stably integrated the plasmid into their chromosomes; the selected cells grow to form foci, which, in turn, can be cloned and expanded into cell lines.

A number of selection systems may be used, including but not limited to, the herpes simplex virus thymidine kinase (Wigler, Cell 11 (1977), 223), hypoxanthine-guanine phosphoribosyltransferase (Szybalska, Proc. Natl. Acad. Sci. USA 48 (1962), 2026), and adenine phosphoribosyltransferase (Lowy, Cell 22 (1980), 817) in tk-, hgprt-or aprt-cells, respectively. Also, antimetabolite resistance can be used as the basis of selection for dhfr, which confers resistance to methotrexate (Wigler, Proc. Natl. Acad. Sci. USA 77 (1980), 3567; O'Hare, Proc. Natl. Acad. Sci. USA 78 (1981), 1527), gpt, which confers resistance to mycophenolic acid (Mulligan, Proc. Natl. Acad. Sci. USA 78 (1981), 2072); neo, which confers resistance to the aminoglycoside G-418 (Colberre-Garapin, J. Mol. Biol. 150 (1981), 1); hygro, which confers resistance to hygromycin (Santerre (1984) Gene 30:147); or puromycin (pat, puromycin N-acetyl transferase). Additional selectable genes have been described, e.g., trpB, which allows cells to utilize indole in place of tryptophan, hisD, which allows cells to utilize histinol in place of histidine (Hartman, Proc. Natl. Acad. Sci. USA 85 (1988), 8047); and ODC (ornithine decarboxylase) which confers resistance to the ornithine decarboxylase inhibitor, 2-(difluoromethyl)-DL-ornithine, DFMO (McCologue, 1987, In: Current Communications in Molecular Biology, Cold Spring Harbor Laboratory ed.).

Uses and Methods of Administration

In the methods of the inventions, a soluble BAFF is administered to a subject to treat a Th2-mediated condition. The subject treated is a mammal, e.g., human, monkey, pig, mouse, or rat.

"Administration" is not limited to any particular formulation, delivery system, or route and may include, for example, intrabronchial, parenteral (including subcutaneous, intravenous, intramedullary, intraarticular, intramuscular, or intraperitoneal injection) rectal, topical, transdermal, or oral (for example, in capsules, suspensions, or tablets). Administration may be provided in a single dose or repeatedly, and in any of a variety of pharmaceutical compositions containing physiologically acceptable salt forms, and/or with an acceptable pharmaceutical excipients. Physiologically acceptable salt forms and pharmaceutical formulations and excipients are known (see, e.g., 2004 Physicians' Desk Reference® (PDR) (2003) Thomson Healthcare, 58th ed; Gennado et al., (2000), 20th ed, Lippincott, Williams & Wilkins) Remington: The Science and Practice of Pharmacy.

A number of therapeutic agents are useful in the management and treatment of asthma. These include, but are not limited to, bronchodilators, e.g., anticholinergic bronchodilators to relax the airway (e.g., ipratropium bromide, albuterol/ipratropium bromide); beta agonists to relax airway muscles (e.g., epinephrine, metaproterenol, terbutaline, isoetharinemesylate, isoetharine, isuprel, pirbuterol, albuterol, salmeterol, bitolterol); oral or inhaled corticosteroids to reduce inflammation (e.g., hydrocortisone, cortisone, dexamethasone, prednisolone, prednisone, methylprednisolone, flunisolide, triamcinolone, beclomethosone, dexamethasone, fluticasone, budesonide); leukotriene modifiers to prevent the airways from swelling and blocking airflow and decrease mucus production (e.g., zafirlukast, montelukast sodium, zileuton); and theophylline, which helps, inter alia, to open the airways and reduce release of phlegm. Anti-asthma agents also include therapeutic antibodies (or functional fragments thereof), including, but not limited to, anti-IgE, anti-IL-9, anti-IL-3, anti-IL-4, anti-IL-5, anti-VLA, and anti-migration inhibitory factor (MIF). Soluble BAFF can be administered in combination with one or more of the aforementioned agents to treat allergic asthma.

Therapeutically effective dosages achieved in one animal model may be converted for use in another animal, including humans, using known conversion factors (see, e.g., Freireich et al. (1966) Cancer Chemother. Reports, 50(4):219-244.

### EXAMPLES

**Example 1: BAFF Tg mice exhibit enhanced DTH responses**

Six to eight week old mice were immunised intra-dermally at the base of the tail with mBSA in CFA and challenged 7 days later with a subcutaneous injection of mBSA into the footpad. BAFF Tg mice from 2 distinct transgenic lines (line 1 and line 2) displayed significantly increased footpad swelling compared to WT mice (Fig. 1A). Footpad swelling was comparable in WT and BAFF Tg mice at early time points post-challenge (Fig. 1A), but was significantly higher in BAFF Tg mice at 48 and 72 h post-challenge. DTH responses in BAFF Tg mice at 48 h were also characterised by increased erythema and histological analysis of paw tissue sections revealed an increased cellular infiltrate into the footpads. Serum anti-mBSA Ab isotypes measured 7 days post-footpad challenge showed BAFF Tg mice to have significantly higher titres of mBSA specific IgG1, IgG2a and IgG2b compared to WT mice, while mBSA-specific IgA levels showed a non significant trend towards an increase. Levels of mBSA specific IgM and IgG3 were similar in WT and BAFF Tg mice.

The concentration of BAFF in human blood varies considerably; most individuals express low levels however some autoimmune patients express over 100 ng/ml. BAFF Tg mice also show variable levels of BAFF in blood. To assess the relationship between serum BAFF levels and footpad swelling, serum from both WT and BAFF Tg mice was collected 48 h after challenge and the level of BAFF in serum was determined by ELISA. Both lines of BAFF Tg mice displayed high levels of BAFF in serum (line 1 average concentration 215.03 ng/ml ± 50.69, line 2 average concentration 1128.2 ng/ml ± 315.8), whereas lower levels of BAFF were detected in WT mice (5.27 ng/ml ± 1.47), similar to levels in unimmunised mice (34). When the level of BAFF in the serum was correlated with footpad swelling for each mouse (Fig. 1B), a strong correlation was observed (r2 = 0.593). Thus systemic BAFF levels may regulate the magnitude of certain T cell responses.

We next examined DTH responses in BAFF deficient mice. These mice were competent in mounting a normal DTH response (Fig. 2A). Also, WT mice treated with BCMA-Ig showed no significant reduction in DTH responses compared to untreated mice (Fig. 2B). Thus BAFF is not essential for basal DTH responses, but in circumstances of BAFF over-production, the magnitude of DTH responses correlate with levels of BAFF.

The mechanisms underlying the enhanced DTH response in BAFF Tg mice were investigated, firstly by examining T cell responses to mBSA in vitro. WT and BAFF Tg mice were immunised at the tailbase as described above, and inguinal lymph nodes were collected 7 days later. Lymph node cells were restimulated in vitro with mBSA for 72 h. T cells from BAFF Tg mice gave a significantly stronger recall response compared to WT mice, displaying a 3-fold increase in proliferation (Fig. 3A), and a 10-fold higher IFN-gamma production in culture supernatants (Fig. 3B). IL-4 and IL-5 levels were below the level of detection in both WT and BAFF Tg mice. Thus in an environment of high BAFF levels, T cell responses to antigen are augmented.

The numbers of effector memory T cells are expanded in BAFF Tg mice, but not in µMT x BAFF Tg mice (data not shown). Thus, the altered makeup of the T cell compartment in BAFF Tg mice is dependent on B cells. BAFF-mediated changes in B cell numbers or function affect T cell subset composition and responses in the DTH reaction.

**Example 2: BAFF Tg mice have compromised allergic airway responses**

The response of WT and BAFF Tg mice were examined in a Th2-driven OVA model of allergic airway inflammation. Measurement of cell numbers in the BAL fluid showed that exposure of WT mice to OVA aerosol resulted in substantial eosinophil infiltration (Fig. 4A), which is characteristic for this model (39). In contrast, BAFF Tg mice showed a significant reduction in eosinophil infiltration. In accordance with BAL fluid cell numbers, histochemical staining of lung tissue sections revealed greatly reduced numbers of peribronchial and perivascular leukocytes in BAFF Tg mice compared to WT controls.

Lymphocytes from peri-bronchial lymph nodes were collected after the final aerosol exposure, and restimulated in vitro with OVA for 72 h. WT lymphocytes showed a strong increase in proliferation in response to restimulation with OVA, in contrast to lymphocytes from BAFF Tg mice (Fig. 4B). Cytokine measurements showed that, after restimulation with OVA, WT lymph nodes cells produced high levels of IL-5, while cells from BAFF Tg mice showed 10-fold reduced levels (Fig. 4C). In addition, small but demonstrable levels of IFN-gamma were detected in some BAFF Tg cultures, although this result was not always reproducible. Of note, there was no compensatory increase in IL-10 production in BAFF Tg cultures (not shown). Thus, BAFF Tg mice show a suppression of Th2-mediated allergic airway inflammation, and an associated reduction in lymph node T cell proliferation and IL-5 production in response to OVA challenge.

To determine whether initial priming defects were responsible for the observed reduction in airway eosinophilia in BAFF Tg mice we performed immunisation and in vitro recall experiments. Mice were immunised at the tailbase with OVA/alum mix, and inguinal lymph nodes were removed after 7 days and restimulated in vitro for 72 h. As expected, WT lymph node cells showed increased proliferation upon stimulation with OVA. BAFF Tg lymph node cells showed an approximately 3-fold increase in proliferation over WT mice (Fig. 5A). In addition, cytokine ELISA from supernatants of restimulated cultures from BAFF Tg mice demonstrated significantly increased levels of IL-5 and IFN-gamma compared to WT mice (Fig. 5B). Thus BAFF Tg mice have increased OVA-specific recall responses, and the defect in lung eosinophilia observed in BAFF Tg mice was not due to impaired priming to OVA.

The results that µMT x BAFF Tg mice were protected from the augmented DTH responses seen in BAFF Tg mice (data not shown) led to the question whether BAFF-mediated suppression of airway inflammation would be relieved in µMT x BAFF Tg mice. Differential cell counts from BAL fluid showed no significant differences in cell numbers between WT and µMT mice in response to OVA aerosol, with characteristic eosinophil infiltration observed in either case (Fig. 6A). As for BAFF Tg mice, µMT x BAFF Tg mice showed a significant reduction in the eosinophil infiltration in response to OVA challenge. Restimulation with OVA of peri-bronchial lymph node T cells from WT and µMT mice showed strong induction of proliferation (Fig. 6B) and IL-5 production (Fig. 6C), whereas there was strong suppression of proliferation and IL-5 production in µMT x BAFF Tg mice (Fig. 6B and C), as we saw in BAFF Tg mice. There were no detectable levels of IFN-gamma or IL-10 in any of the cultures. These results demonstrate that BAFF-mediated suppression of Th2-dependent allergic airway inflammation occurs by mechanisms that are B cell-independent.

The specification is most thoroughly understood in light of the teachings of the references cited within the specification. The embodiments within the specification provide an illustration of embodiments of the invention and should not be construed to limit the scope of the invention. The skilled artisan readily recognizes that many other embodiments are encompassed by the invention. All publications, patents, and biological sequences cited in this disclosure are incorporated by reference in their entirety. To the extent the material incorporated by reference contradicts or is inconsistent with the present specification, the present specification will supersede any such material. The citation of any references herein is not an admission that such references are prior art to the present invention.

Unless otherwise indicated, all numbers expressing quantities of ingredients, cell culture, treatment conditions, and so forth used in the specification, including claims, are to be understood as being modified in all instances by the term "about." Accordingly, unless otherwise indicated to the contrary, the numerical parameters are approximations and may vary depending upon the desired properties sought to be obtained by the present invention. Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the following claims.

## Claims

1. A soluble BAFF polypeptide for the treatment of a Th2-mediated disorder in a mammal.

2. Use of a soluble BAFF polypeptide for the preparation of a pharmaceutical composition for the treatment of a Th2-mediated disorder in a mammal.

3. The soluble BAFF polypeptide according to claim 1, or the use according to claim 2, wherein the mammal is human.

4. The soluble BAFF polypeptide according to claim 1, or the use according to claim 2, wherein the disorder is atopy or allergic asthma.

5. The soluble BAFF polypeptide according to any one of the claims 1, 3 or 4, or the use according to any one of the claims 2 to 4, wherein the soluble BAFF polypeptide comprises:
(a) a fragment of a BAFF extracellular domain capable of binding to a BAFF receptor; or
(b) a substantial part of the TNF-like domain of BAFF; and does not comprise the BAFF intracellular or transmembrane domains.

6. The soluble BAFF polypeptide according to claim 5, or the use according to claim 5, wherein the soluble BAFF polypeptide comprises
(a) amino acids 145-284 of SEQ ID NO:1;
(b) amino acids 169-308 of SEQ ID NO:2; or
(c) amino acids 11-150 of SEQ ID NO:3.

7. The soluble BAFF polypeptide according to claim 5, or the use according to claim 5, wherein the soluble BAFF polypeptide comprises an Fc portion of an immunoglobulin.

8. The soluble BAFF polypeptide according to claim 5, or the use according to claim 5, wherein the soluble BAFF polypeptide is administered in combination with a second therapeutic agent for the disorder.

9. The soluble BAFF polypeptide according to claim 5, or the use according to claim 5, wherein the soluble BAFF polypeptide is administered at a dosage between 0.005 and 5 mg/kg/day.

10. The soluble BAFF polypeptide according to claim 5, or the use according to claim 5, wherein the soluble BAFF polypeptide comprises a polypeptide at least 90% identical to amino acids 145-284 of SEQ ID NO:1.

11. The soluble BAFF polypeptide according to claim 5, or the use according to claim 5, wherein the soluble BAFF polypeptide comprises:
(a) amino acids 145-284 of SEQ ID NO:1;
(b) amino acids 170-308 of SEQ ID NO:2;
(c) amino acids 11-150 of SEQ ID NO:3;
(d) a sequence at least 70% identical to any one of (a) - (c);
(e) a fragment of BAFF extracellular domain capable of binding to a BAFF receptor;
(f) substantial part of the TNF-like domain of BAFF; or
(g) any one of (a) - (e) fused to an Fc portion of an immunoglobulin.

12. A soluble BAFF polypeptide for the treatment of a subject who has an inadequate Th1 response.

13. Use of a soluble BAFF polypeptide for the preparation of a pharmaceutical composition for the treatment of a subject who has an inadequate Th1 response.

14. The soluble BAFF polypeptide according to claim 12, or the use according to claim 13, wherein the subject is infected with a viral or intracellular bacterial pathogen.

## Patentansprüche

1. Lösliches BAFF-Polypeptid zur Behandlung einer Th2-vermittelten Krankheit in einem Säuger.

2. Verwendung eines löslichen BAFF-Polypeptids für die Herstellung eines Arzneimittels zur Behandlung einer Th2-vermittelten Krankheit in einem Säuger.

3. Lösliches BAFF-Polypeptid nach Anspruch 1 oder Verwendung nach Anspruch 2, wobei der Säuger ein Mensch ist.

4. Lösliches BAFF-Polypeptid nach Anspruch 1 oder Verwendung nach Anspruch 2, wobei die Krankheit Atopie oder allergisches Asthma ist.

5. Lösliches BAFF-Polypeptid nach einem der Ansprüche 1, 3 oder 4 oder Verwendung nach einem der Ansprüche 2 bis 4, wobei das lösliche BAFF-Polypeptid umfasst:
(a) ein Fragment einer extrazellulären Domäne von BAFF, das fähig ist, an einen BAFF-Rezeptor zu binden; oder
(b) einen wesentlichen Teil der TNF-ähnlichen Domäne von BAFF, der nicht die intrazellulären oder transmembranen Domänen von BAFF umfasst.

6. Lösliches BAFF-Polypeptid nach Anspruch 5 oder Verwendung nach Anspruch 5, wobei das lösliche BAFF-Polypeptid umfasst:
(a) Aminosäuren 145-284 der SEQ ID NO: 1;
(b) Aminosäuren 169-308 der SEQ ID NO: 2; oder
(c) Aminosäuren 11-150 der SEQ ID NO: 3.

7. Lösliches BAFF-Polypeptid nach Anspruch 5 oder Verwendung nach Anspruch 5, wobei das lösliche BAFF-Polypeptid einen Fc-Anteil eines Immunglobulins umfasst.

8. Lösliches BAFF-Polypeptid nach Anspruch 5 oder Verwendung nach Anspruch 5, wobei das lösliche BAFF-Polypeptid in Kombination mit einem zweiten Therapeutikum für die Krankheit verabreicht wird.

9. Lösliches BAFF-Polypeptid nach Anspruch 5 oder Verwendung nach Anspruch 5, wobei das lösliche BAFF-Polypeptid in einer Dosierung zwischen 0.005 und 5 mg/kg/Tag verabreicht wird.

10. Lösliches BAFF-Polypeptid nach Anspruch 5 oder Verwendung nach Anspruch 5, wobei das lösliche BAFF-Polypeptid ein Polypeptid umfasst, das mindestens zu 90% identisch zu den Aminosäuren 145-284 der SEQ ID NO: 1 ist.

11. Lösliches BAFF-Polypeptid nach Anspruch 5 oder Verwendung nach Anspruch 5, wobei das lösliche BAFF-Polypeptid umfasst:
(a) Aminosäuren 145-284 der SEQ ID NO: 1;
(b) Aminosäuren 170-308 der SEQ ID NO: 2;
(c) Aminosäuren 11-150 der SEQ ID NO: 3;
(d) eine Sequenz, die mindestens zu 70% identisch zu einem der Polypeptide nach (a)-(c) ist;
(e) ein Fragment der extrazellulären Domäne von BAFF, die fähig ist, an den BAFF-Rezeptor zu binden;
(f) einen wesentlichen Teil der TNF-ähnlichen Domäne von BAFF; oder
(g) eines der Polypeptide nach (a)-(e) fusioniert an einen Fc-Anteil eines Immunglobulins.

12. Lösliches BAFF-Polypeptid zur Behandlung eines Individuums, das eine unzureichende Th1-Antwort hat.

13. Verwendung eines löslichen BAFF-Polypeptids für die Herstellung eines Arzneimittels zur Behandlung eines Individuums, das eine unzureichende Th1-Antwort hat.

14. Lösliches BAFF-Polypeptid nach Anspruch 12 oder Verwendung nach Anspruch 13, wobei das Individuum mit einem viralen oder intrazellulären bakteriellen Pathogen infiziert ist.

## Revendications

1. Polypeptide soluble BAFF pour le traitement d'un trouble induit par TH2 chez un mammifère.

2. Utilisation d'un polypeptide soluble BAFF pour la préparation d'une composition pharmaceutique pour le traitement d'un trouble induit par TH2 chez un mammifère.

3. Polypeptide soluble BAFF selon la revendication 1, ou utilisation selon la revendication 2, le mammifère étant un humain.

4. Polypeptide soluble BAFF selon la revendication 1, ou utilisation selon la revendication 2, le trouble étant une atopie ou un asthme allergique.

5. Polypeptide soluble BAFF selon l'une quelconque des revendications 1, 3 ou 4, ou utilisation selon l'une quelconque des revendications 2 à 4, le polypeptide soluble BAFF comprenant :
(a) un fragment d'un domaine extracellulaire de BAFF capable de se lier à un récepteur BAFF ; ou
(b) une partie substantielle du domaine de BAFF apparenté à TNF ; et ne comprend pas les domaines intracellulaire ou transmembranaire de BAFF.

6. Polypeptide soluble BAFF selon la revendication 5, ou utilisation selon la revendication 5, le polypeptide soluble BAFF comprenant
(a) les acides aminés 145-284 de la SEQ ID NO :1 ;
(b) les acides aminés 169-308 de la SEQ ID NO :2 ; ou
(c) les acides aminés 11-150 de la SEQ ID NO :3.

7. Polypeptide soluble BAFF selon la revendication 5, ou utilisation selon la revendication 5, le polypeptide soluble BAFF comprenant une partie Fc d'une immunoglobuline.

8. Polypeptide soluble BAFF selon la revendication 5, ou utilisation selon la revendication 5, le polypeptide soluble BAFF étant administré en combinaison avec un second agent thérapeutique pour le trouble.

9. Polypeptide soluble BAFF selon la revendication 5, ou utilisation selon la revendication 5, le polypeptide soluble BAFF étant administré à un dosage compris entre 0,005 et 5 mg/kg/jour.

10. Polypeptide soluble BAFF selon la revendication 5, ou utilisation selon la revendication 5, le polypeptide soluble BAFF comprenant un polypeptide au moins à 90% identique aux acides aminés 145-284 de la SEQ ID NO :1.

11. Polypeptide soluble BAFF selon la revendication 5, ou utilisation selon la revendication 5, le polypeptide soluble BAFF comprenant :
(a) les acides aminés 145-284 de la SEQ ID NO :1 ;
(b) les acides aminés 170-308 de la SEQ ID NO :2 ;
(c) les acides aminés 11-150 de la SEQ ID NO :3 ;
(d) une séquence au moins à 70% identique à l'une quelconque de (a) à (c) ;
(e) un fragment d'un domaine extracellulaire de BAFF capable de se lier à un récepteur BAFF ;
(f) une partie substantielle du domaine de BAFF apparenté à TNF ; ou
(g) l'un quelconque de (a) à (e) fusionné à une partie Fc d'une immunoglobuline.

12. Polypeptide soluble BAFF pour le traitement d'un sujet qui a une réponse Th1 inadéquate.

13. Utilisation d'un polypeptide soluble BAFF pour la préparation d'une composition pharmaceutique pour le traitement d'un sujet qui a une réponse Th1 inadéquate.

14. Polypeptide soluble BAFF selon la revendication 12, ou utilisation selon la revendication 13, le sujet étant infecté par un pathogène viral ou par un pathogène intracellulaire bactérien.
